Europäisches Patentamt

European Patent Office

Office européen des brevets

(11) Publication number: **0 443 735 A1**

## EUROPEAN PATENT APPLICATION

(21) Application number: 91300788.6

(51) Int. Cl.⁵: **A61M 5/32**

(22) Date of filing: 31.01.91

(30) Priority: 17.02.90 GB 9003643

(43) Date of publication of application:
28.08.91 Bulletin 91/35

(84) Designated Contracting States:
DE FR SE

(71) Applicant: Smiths Industries Public Limited
Company
765, Finchley Road
London, NW11 8DS(GB)

(72) Inventor: Lester, Graham George
Heronsgate, Burmash Road
Hythe, Kent, CT21 4NH(GB)

(74) Representative: Flint, Jonathan McNeill
SMITHS INDUSTRIES PUBLIC LIMITED
COMPANY 765 Finchley Road
London NW11 8DS(GB)

(54) **Needle guards and assemblies.**

(57) A guard (3) for a needle (1) is an extruded resilient channel, larger than the needle, with a recess (30) of circular section and an opening (31) formed by two flared wings (32, 33). The outer width of the opening (31) is at least twice the width of the recess (30) so that a needle (1) may be laterally snapped into the guard (3). The diameter of the recess (30) is less than the external diameter of the needle (1) such that the guard (3) grips the needle (1).

Fig. 3

EP 0 443 735 A1

This invention relates to needle guards and to assemblies including such guards.

In order to protect needles used in medico-surgical instruments and apparatus, a guard in the form of a sleeve of rigid plastics material is pushed over the needle onto the needle hub. The internal diameter of the sleeve is selected so that it is a push fit on the hub and can readily be removed when the needle is used.

These guards suffer from several disadvantages. Firstly, insertion of the needle into the end of the sleeve is difficult because of the relatively small internal diameter of the sleeve. This can result in damage to the point of the needle and wounding of the user. If an attempt is made to replace a guard after use, this can result in cross infection of the user, if he should scratch himself with the needle because of incorrect insertion. Secondly, where the needle has a bent tip, such as with Tuohy needles, there is a tendency for the tip of the needle to scrape the inside of the guard on insertion which can lead to material of the guard entering the needle. Furthermore, there are needles which do not have a hub that is suitable for receiving a sleeve of this kind.

It is an object of the present invention to provide a needle guard and an assembly that can be used to avoid these disadvantages.

According to one aspect of the present invention there is provided a needle guard for use in conjunction with a needle, characterised in that the guard is in the form of an elongate channel member of resilient material, that the channel member has a recess and an elongate opening to the recess along the major part of the length of the guard, and that the opening is flared radially from a width adjacent the recess that is less than the diameter of the needle to an outer width greater than the diameter of the needle, such that a needle can be pushed laterally into the guard through the opening and that the resilience of the guard will grip the needle when inserted.

The recess is preferably of substantially circular section. The outer width of the opening may be at least twice the width of the recess. The portion of the opening flared radially may form two wings inclined at 90 degrees to one another. The guard is preferably of a resilient plastics material and may be a plastics extrusion.

According to another aspect of the present invention there is provided a needle assembly characterised in that the needle projects from a laterally extending end face of a hub and that the needle guard abuts the end face.

A medico-surgical needle assembly including a guard, in accordance with the present invention, will now be described, by way of example, with reference to the accompanying drawings, in which:

Figure 1 is a sectional side-elevation of the assembly;

Figure 2 is a plan view of the assembly;

Figure 3 is an enlarged transverse section along line III-III of Figure 2;

Figure 4 illustrates a method of assembly; and

Figure 5 shows a part of an alternative assembly.

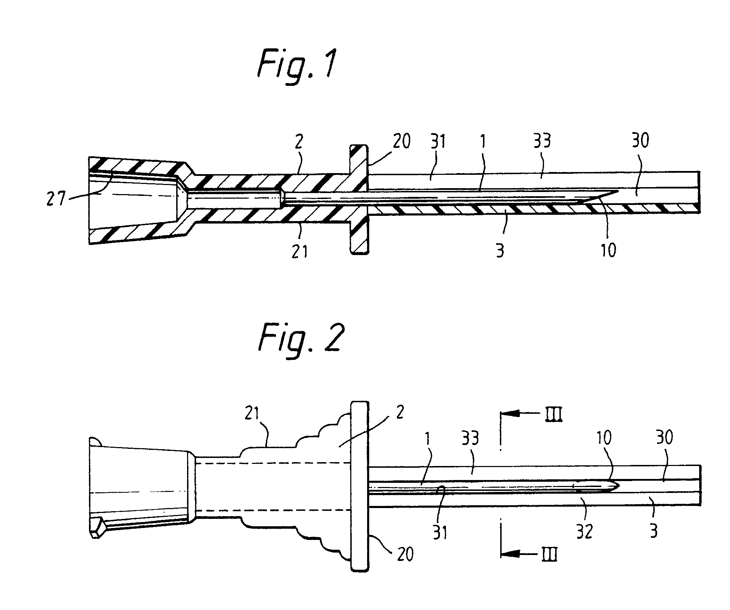

With reference to Figures 1 to 3, the assembly comprises a hollow metal needle 1 joined at its rear end to a moulded plastics hub 2 and a needle guard 3 that protects the needle.

The needle 1 and hub 2 are of conventional construction, of the kind described in GB 2225953A. The needle 1 is 16 Gauge, with an external diameter of 1.651mm, and is 17mm long from the hub 2. At its forward end 10 the needle 1 is formed with a sharply pointed bevelled tip.

The hub 2 is approximately the same length as the needle 1. At its forward, patient end, the hub 2 has a laterally extending flat face 20 which is substantially rectangular in section but with its longer edges curved convexly. The face 20 is about 14mm long and 10mm wide at its widest point giving it a surface area of between 100-150mm compared with the cross sectional area of the needle 1 which is about 2mm .

The intermediate portion 21 of the hub 2, extending rearwardly from the face 20, provides a gripping region by which the hub can be gripped between finger and thumb. At its rear, machine end the hub 2 has a female bore 27 which, in use receives the nose of a syringe (not shown).

The needle guard 3 is one-piece unitary extrusion or moulding of natural low-density polyethylene or a similar resilient plastics material. The guard 3 is in the form of an elongate channel 37mm long, with a central circular recess 30 that extends the length of guard. The recess 30 has a diameter of 1.63mm, that is, slightly smaller than the external diameter of the needle 1. An elongate opening 31 opens into the recess 30 and extends the entire length of the guard. The opening 31 is provided between two wings 32 and 33 which are inclined at 90 degrees to one another and which have rounded ends 34 and 35. The opening 31 formed between the two wings, therefore, tapers in width, being about 4mm wide at its outer edge, and reducing to a width less than 1.5mm at its narrowest, where it opens into the recess 30. At its widest, therefore, the opening 31 is wider than the needle 1 (preferably being at least twice the width of the needle and the diameter of the recess), whereas at its narrowest, it is narrower than both the recess 30 and the needle.

When assembled on the needle 1, the machine end of the guard 3 abuts the face 20 of the hub and its patient end projects beyond the tip 3 of the needle 1. Because the central recess 30 is smaller in diameter than the needle 1, the guard will be sprung outwardly slightly and exert a resilient gripping force about the needle. This is sufficient to retain the guard on the needle but allows it to be pulled off readily before the needle is used. Other resilient materials could be used such as metal, which would provide a similar resilient, spring grip on the needle. It can be seen that the guard 3 does not rely on engagement with the needle hub 2 to retain it on the needle 1.

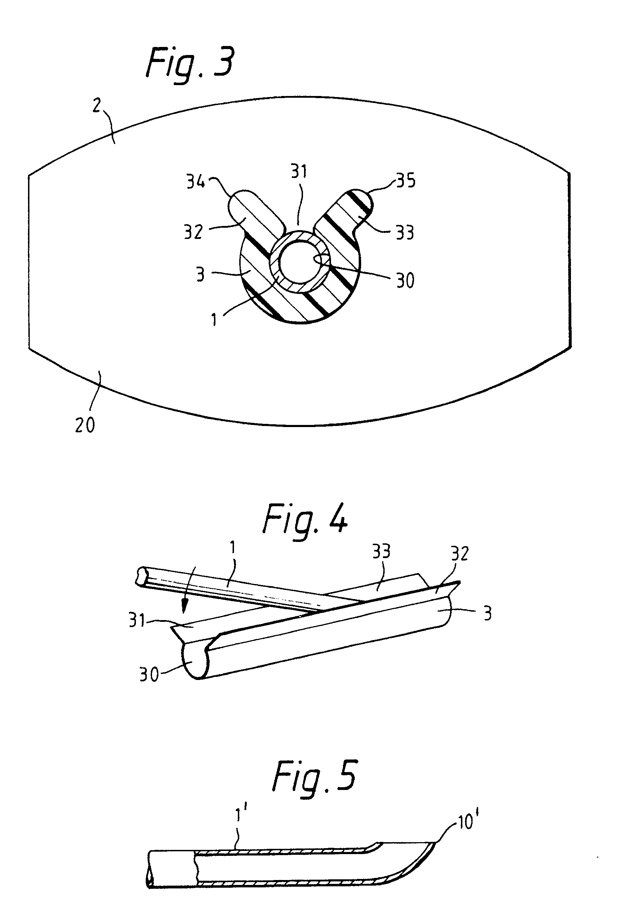

The guard 3 can be assembled on the needle 1 in several different ways. It can be pushed on the needle axially, in the way necessary with conventional guards. Alternatively, the guard 3 can be pushed laterally on the needle by locating the needle to extend parallel to the opening 31, between the two wings 32 and 33, and then pushing it into the recess 30 as a snap fit. The third way of assembly is illustrated in Figure 4. This involves angling the needle 1 to the guard 3 at about 45 degrees and pushing the tip of the needle laterally between the wings 32 and 33 into the recess 30. The needle 1 is then pivoted down about its tip 10 until the rear part of the needle snaps into the recess 30.

The latter two methods of assembly reduce the risk of the user being wounded by the tip of the needle because it is easier to locate the needle between the wings 32 and 33 than to insert it axially, and because the user's fingers can be kept clear of the needle tip. These assembly techniques are also more suited to use with needles 1' having a bent tip 10', such as of the kind shown in Figure 5. This is because there need be no sliding movement of the tip on entry to the guard, thereby reducing the risk of guard material being pared off the inside of the guard by the tip of the needle.

It will be appreciated that the recess need not be of circular section but may, for example, be triangular or of other polygonal shape the recess being sized to grip the needle when assembled. The recess may be larger than the outer diameter of the needle if it is provided with ribs, or other projections, which grip the needle when inserted.

It will also be appreciated that the guard could be used with other medico-surgical needles.

## Claims

1. A needle guard for use in conjunction with a needle, characterised in that the guard (3) is in the form of an elongate channel member of resilient material, that the channel member has a recess (30) and an elongate opening (31) to the recess (30) along the major part of the length of the guard (3), and that the opening (31) is flared radially from a width adjacent the recess (30) that is less than the diameter of the needle (1) to an outer width greater than the diameter of the needle (1), such that a needle (1) can be pushed laterally into the guard (3) through the opening and that the resilience of the guard (3) will grip the needle (1) when inserted.

2. A needle guard according to Claim 1, characterised in that the recess (30) is of substantially circular section.

3. A needle guard according to Claim 1 or 2, characterised in that the outer width of the opening (31) is at least twice the width of the recess (30).

4. A needle guard according to any one of the preceding claims, characterised in that the portion of the opening (31) flared radially forms two wings (32 and 33) inclined at 90 degrees to one another.

5. A needle guard according to any one of the preceding claims, characterised in that the guard (3) is of a resilient plastics material.

6. A needle guard according to Claim 5, characterised in that the guard (3) is a plastics extrusion.

7. A needle assembly including a needle and a needle guard according to any one of the preceding claims, characterised in that the needle guard (3) is longer than the needle (1) and that the guard (3) is mounted on and protects the needle (1).

8. A needle assembly according to Claim 7, characterised in that the needle (1) projects from a laterally extending end face (20) of a hub, and that the needle guard (3) abuts the end face (20).

## *Fig.1*

## *Fig. 2*

4

# Fig. 3

# Fig. 4

# Fig. 5

## DOCUMENTS CONSIDERED TO BE RELEVANT

EP 91300788.6

| Category | Citation of document with indication, where appropriate, of relevant passages | Relevant to claim | CLASSIFICATION OF THE APPLICATION (Int. Cl.5) |
|---|---|---|---|
| Y | US - A - 3 537 452 (G.W. WILKS)     * Totality * | 1-3,5, 7 | A 61 M 5/32 |
| A | | 4,6,8 | |
| Y | US - A - 4 883 469 (S.C. GLAZIER)     * Fig. 1-5; column 5, line 53 - column 6, line 5; abstract * | 1-3,5, 7 | |
| A | US - A - 4 643 722 (W.I. SMITH)     * Fig. 9,10,19; column 5, lines 34-45, 53-58; abstract * | 1,2,4, 5,7,8 | |
| A | GB - A - 1 233 302 (BAXTER LAB.)     * Totality * | 1,5,6, 7 | |
| | | | **TECHNICAL FIELDS SEARCHED (Int. Cl.5)** |
| | | | A 61 M |

The present search report has been drawn up for all claims

| Place of search | Date of completion of the search | Examiner |
|---|---|---|
| VIENNA | 27-05-1991 | LUDWIG |

EPO FORM 1503 03.82 (P0401)